# EUROPÄISCHE PATENTSCHRIFT

(11) **EP 1 158 940 B1**
(45) Veröffentlichungstag und Bekanntmachung des Hinweises auf die Patenterteilung: **17.11.2004**
(21) Anmeldenummer: 01900127.0
(22) Anmeldetag: 05.01.2001
(51) Int. Cl.: A61F 2/68

(54) **VORRICHTUNG UND VERFAHREN ZUR FERNWARTUNG EINER ELEKTRONISCH ANSTEUERBAREN PROTHESE**
DEVICE AND METHOD FOR THE REMOTE SERVICING OF AN ELECTRONICALLY CONTROLLED PROSTHESIS
DISPOSITIF ET PROCEDE DE TELE-ENTRETIEN D'UNE PROTHESE COMMANDEE ELECTRONIQUEMENT

(30) Priorität: 11.01.2000 DE 10000781
(43) Veröffentlichungstag der Anmeldung: 05.12.2001
(73) Patentinhaber: Biedermann Motech GmbH, 78054 Villingen-Schwenningen (DE)
(72) Erfinder: BIEDERMANN, Lutz, 78048 VS-Villingen (DE); MATTHIS, Wilfried, 79367 Weisweil (DE); SCHULZ, Christian, 09648 Mittweida (DE)
(74) Vertreter: Prüfer, Lutz H., Dipl.-Phys.
(86) Internationale Anmeldenummer: PCT/EP2001/000078
(87) Internationale Veröffentlichungsnummer: WO 2001/050986

(56) Entgegenhaltungen:
- DE-A- 19 754 690
- DE-A- 19 859 931
- GB-A- 2 328 160
- US-A- 5 413 611
- US-A- 5 443 524
- US-A- 5 484 389

## Beschreibung

Die Erfindung betrifft die Vorrichtung und ein Verfahren zur Fernwartung einer elektronisch ansteuerbaren Prothese.

Elektronisch gesteuerte Prothesen sind beispielsweise in Form von Beinprothesen mit einem künstlichen Kniegelenk aus der Med. Orth. Tech. 117 (1997), Seiten 31 bis 35 bekannt. Bei diesen Prothesen werden die Steuerparameter in Abhängigkeit von eine Bewegung des Prothesenträgers charakterisierenden Bewegungsdaten ermittelt und programmiert. Zum Einstellen der Steuerparameter für ein optimales Gangbild ist es jedoch erforderlich, dass ein Orthopädietechniker den die Prothese tragenden Patienten beim Gehen beobachtet und die Steuerparameter in Abhängigkeit von seiner Beobachtung einstellt. Die Prothese wird anschließend mit den bei der Einstellung ermittelten optimalen Steuerdaten angesteuert.

Es besteht das Problem, daß während des täglichen Betriebs der Prothese Veränderungen am Gehverhalten des Prothesenträgers auftreten können, beispielsweise ändert sich das Gangbild aufgrund Veränderung des Gewichts des Protesenträgers oder des Tragens anderer Schuhe etc.. Die Prothese ist dann nicht mehr optimal eingestellt und der Prothesenträger muß, um angenehm gehen zu können, eine Neueinstellung durch den Orthopädietechniker vornehmen lassen. Dies ist mit Zeitaufwand verbunden und beschwerlich für den Prothesenträger.

Es ist Aufgabe der Erfindung eine Vorrichtung und ein Verfahren bereitzustellen, mit der bzw. mit dem die Wartung und das Nacheinstellen einer elektronisch gesteuerten Prothese vereinfacht wird.

Die Aufgabe wird gelöst durch eine Vorrichtung nach Patentanspruch 1 und ein Verfahren nach Patentanspruch 7. Weiterbildungen der Erfindung sind in den Unteransprüchen angegeben.

Weitere Merkmale und Zweckmäßigkeiten der Erfindung ergeben sich aus der Beschreibung eines Ausführungsbeispieles anhand der Figuren. Von den Figuren zeigen:
- Fig. 1: eine schematische Darstellung einer Beinprothese mit einem künstlichen Kniegelenk und einer dazugehörigen Steuerung; und
- Fig. 2: eine schematische Darstellung einer erfindungsgemäßen Vorrichtung zur Fernwartung einer elektronisch ansteuerbaren Prothese.

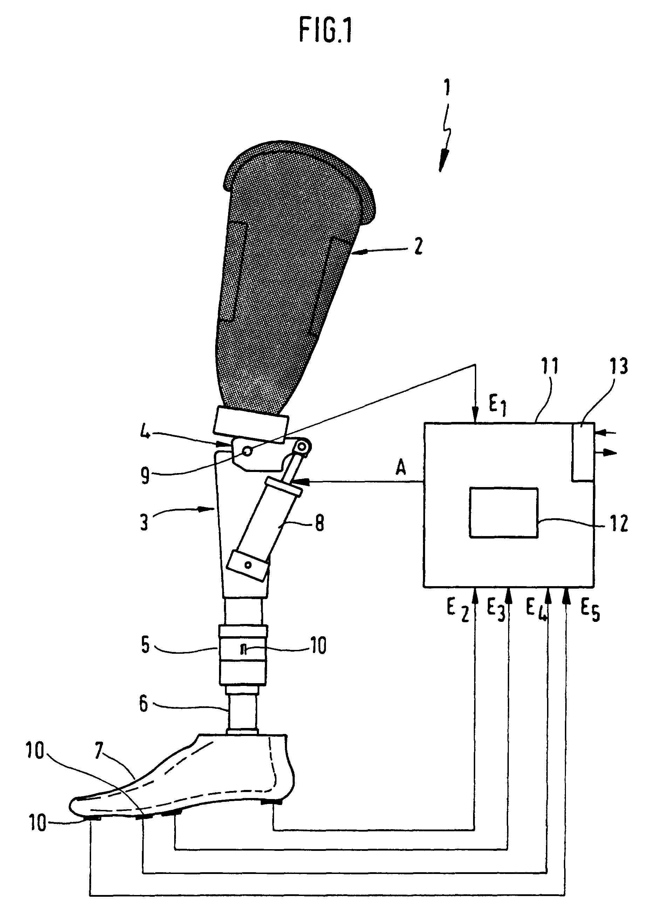

Wie aus Fig. 1 ersichtlich ist, umfaßt die Prothese 1 in bekannter Weise ein Oberschenkelteil 2 und ein Unterschenkelteil 3 und ein die beiden verbindendes Kniegelenk 4. Das Unterschenkelteil 3 weist ein Schienbeinteil 5 mit einem Unterschenkelrohr 6 und ein mit diesem verbundenes Fußteil 7 auf.

In dem Fußteil 7 ist eine in der Figur nicht dargestellte Blattfeder zum Ermöglichen eines federnden Auftrittes vorgesehen. Das Kniegelenk 4 umfaßt ein Dämpfungselement in Form einer hydraulischen Kolben-Zylindereinrichtung 8 auf. Die Beinprothese weist ferner eine Anzahl von Sensoren zur Bewegungs- und Kraftmessung auf. Im Kniegelenk 4 ist ein Kniewinkelsensor zur Erfassung des Kniewinkels bei der Bewegung des Prothesenträgers vorgesehen. Im Bereich der Fußsohle oder alternativ am Schienbeinteil 5 oder dem Unterschenkelrohr 6 sind Kraftsensoren 10 zur Erfassung der Kräfte, die beim Gehen mit der Prothese wirken, vorgesehen. Je nach Zweckmäßigkeit sind gegebenenfalls im Schienbeinteil 5 Beschleunigungssensoren angebracht.

Zur Steuerung des Flexionsverhaltens des Kniegelenks 4 ist eine Steuervorrichtung 11 vorgesehen, welche eine Mehrzahl von Eingängen E₁, E₂, E₃, E₄ zum Empfangen der Signale der beschriebenen Sensoren aufweist. Ferner beinhaltet die Steuervorrichtung 11 eine (nicht dargestellte) Echtzeituhr sowie eine Datenspeicher- und Mikroprozessoreinheit 12, in der die gemessenen Signale der Sensoren, nachfolgend als Bewegungsdaten bezeichnet, über einen vorbestimmten Zeitraum der Bewegung des Prothesenträgers gespeichert werden und in dem ferner Steuerdaten beispielsweise in Form von Steuerparametern zum Steuern des Betriebs der Prothese abgespeichert sind. Die Steuervorrichtung ist über einen Ausgang A mit der Kolben-Zylindereinrichtung 8 des Kniegelenks verbunden, über den die Steuerdaten an die Kolben-Zylindereinrichtung gesandt werden. Ferner ist die Steuereinrichtung über ein Interface 13 mit einer nachfolgend beschriebenen Vorrichtung zur Fernwartung verbunden. Über das Interface 13 erfolgt ein Austausch von Bewegungsdaten und Steuerdaten mit der Vorrichtung zur Fernwartung. Die Steuervorrichtung 11 ist mit dem Körper des Prothesenträgers verbunden, beispielsweise ist sie in die Prothese selbst integriert oder wird über einen Gürtel am Körper des Prothesenträgers getragen. Die Beinprothese weist ferner nicht dargestellte aufladbare Batterien auf.

Die in der Steuervorrichtung 11 abgelegten Steuerdaten sind bei der erstmaligen Einstellung der Prothese durch einen Orthopädietechniker nach Beobachtung des Gangbildes des Prothesenträgers bestimmt worden.

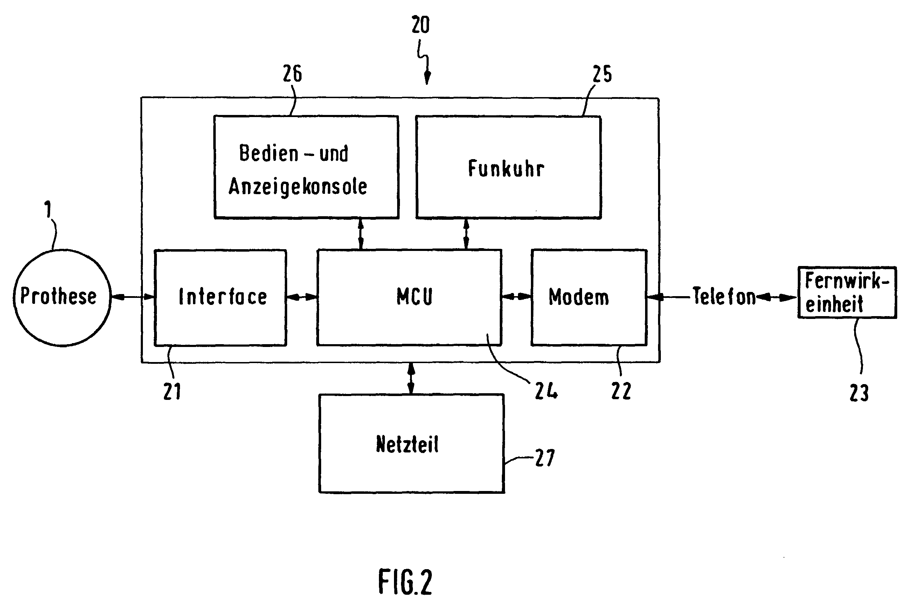

Fig. 2 zeigt eine erfindungsgemäße Fernwartungsvorrichtung 20 bzw. Docking Station zur Fernwartung der in Fig. 1 beschriebenen Beinprothese. Die Fernwartungsvorrichtung 20 erlaubt ein Anschließen der Steuerung 11 der Prothese und das Sammeln von Steuer- und Bewegungsdaten der Prothese, eine Auswertung, Bearbeitung und gegebenenfalls Veränderung der Daten von extern über ein Datenfernübertragungsnetz und ein Rückübertragen der geänderten Daten an die Steuerung 11 der Prothese. Hierzu weist die Fernwartungsvorrichtung 20 ein Interface 21 auf, über welches die Steuervorrichtung 11 mit der Fernwartungsvorrichtung 20 verbindbar ist und über welches sowohl die Bewegungsdaten, als auch Steuerdaten in beiden Richtung zwischen der Vorrichtung 20 und der Steuervorrichtung 11 nach einem vorbestimmten Datenübertragungsprotokoll übertragbar sind. Ferner ist eine Datenfernübertragungseinrichtung 22 vorgesehen zur Übertragung von Bewegungs- und/oder Steuerdaten an eine externe, sich örtlich entfernt befindliche Fernwirkeinheit 23 über ein Datenfernübertragungsnetz, z.B. das Telephonnetz. Die Datenfernübertragungseinrichtung 22 ist z.B. als Modem oder ISDN-Karte ausgebildet. Die Fernwirkeinheit 23 ist beispielsweise ein Personalcomputer, der seinerseits mit Datenfernübertragungsnetz verbunden ist. Die Fernwartungsvorrichtung 20 beinhaltet ferner eine Microcontrollereinheit (MCU) 24 die als zentrale Steuer- und Datenspeichervorrichtung wirkt. Die MCU steuert das Interface 21 und die Datenfernübertragungseinrichtung 22 und speichert die Bewegungs- und Steuerdaten. Ferner sind in der MCU 24 ein oder mehrere vorbestimmten Programme zum Verarbeiten der Daten abgelegt. Die MCU 24 ist ferner mit einer extern gesteuerten Uhr 25, z.B. einer Funkuhr, verbunden zur Synchronisation der Uhrzeit mit der in der Steuerung 11 der Prothese vorhandenen Echtzeituhr. Es ist ferner eine Bedien- und Anzeigekonsole 26 vorgesehen zum Ermöglichen des Anzeigens von Bewegungs- und/oder Steuerdaten oder Auswertungen derselben und zum Ermöglichen der Eingabe von Befehlen und/oder Daten. Die Vorrichtung zur Fernwartung ist außerdem mit einer Strom- bzw. Spannungsversorgung in Form eines Netzteiles 27 verbunden, welches eine Stand-by-Spannungsversorgung der Prothese 1 erlaubt, wenn diese über das Interface 21 an die Vorrichtung 20 angeschlossen ist und/oder welche das Laden der aufladbaren Batterien der Prothese erlaubt.

Als Fernwartungsvorrichtung 20 ist ein Personalcomputer, der in der häuslichen Umgebung des Prothesenträgers aufstellbar ist, oder ein tragbarer Computer in Form eines Notebooks verwendbar, welches die entsprechenden beschriebenen Elemente aufweist. Die Fernwirkeinheit 23 ist zweckmäßigerweise in der Praxis des Orthopädietechnikers aufgestellt und wird von diesem bedient.

Ein in der MCU 24 abgelegtes Programm erlaubt die Fernwartung der Prothese über die Fernwirkeinheit 23 und das Datenfernübertragungsnetz. Die Funktionsmodule dieses Programms werden nachfolgend anhand des Betriebs beschrieben.

Der Betrieb der Fernwartungsvorrichtung 20 und das erfindungsgemäße Verfahren sind wie folgt: Der Prothesenträger verbindet in seiner häuslichen Umgebung, wenn er die Prothese ablegt, die Steuereinrichtung 11 der Prothese über das Interface 21 mit der Vorrichtung 20 zur Fernwartung. Während die Prothese mit der Vorrichtung 20 verbunden ist, erhält sie Strom über das Netzteil 27 und/oder die Batterien der Prothese werden aufgeladen. Die Steuervorrichtung 11 der Prothese übermittelt sodann die aktuellen Steuerparameter und die über einen vorbestimmten Zeitraum erfaßten Bewegungsdaten an die Vorrichtung 20. Die Kommunikation zwischen der Fernwirkeinheit 23 und der Vorrichtung 20 über ein Datenfernübertragungsnetz wird entweder von dem Prothesenträger selbst durch Eingabe eines entsprechenden Befehls über die Bedien- und Anzeigekonsole 25 initiiert, oder die Fernwartungsvorrichtung 20 beginnt über die MCU 24 gesteuert zu vorbestimmten Zeiten eine Kommunikation mit der Fernwirkeinheit 23. Alternativ kann auch die Fernwirkeinheit 23 zu vorbestimmten Zeiten die Kommunikation mit der Vorrichtung 20 aufnehmen.

Nach dem Herstellen der Verbindung mit der Fernwirkeinheit 23 wird die Prothese anhand vorgegebener Identifikationsdaten erkannt und spezifiziert. Die von der Steuerung der Prothese übertragenen Bewegungs- und Steuerdaten werden in der Vorrichtung 20 gespeichert und einer Vorauswertung unterzogen. Der Orthopädietechniker kann über die Fernwirkeinheit 23 und damit über die Datenfernübertragungseinrichtung 22 auf die in der Vorrichtung 20 gespeicherten Bewegungs- und Steuerdaten zugreifen, diese Daten auswerten und mit diesen Daten vorbestimmte Operationen ausführen und insbesondere die Steuerdaten d.h. die Steuerparameter für die Prothese verändern und somit korrigieren. Solche veränderten oder neuen Steuerdaten werden über das Interface 21 wieder an die Steuervorrichtung 11 der Prothese gegeben.

Das in der MCU 24 abgelegte Fernwartungsprogramm weist folgende Module bzw. Funktionen auf, die zusätzlich zu dem oben beschriebenen Grundprinzip der Datenübertragung von der Prothese in die Vorrichtung und von dort zu einem externen Empfänger und vice versa durchgeführt werden. So kann die Prothese wahlweise in der Vorrichtung 20 über das Netzteil 27 mit Strom versorgt werden und gleichzeitig die Batterien der Prothese für einen nachfolgenden Betrieb aufgeladen werden. Die Bewegungsdaten können vorausgewertet werden, wobei Zeitunterschiede der Prothesenuhr und der Funkuhr mit berücksichtigt werden. Bei Stromausfall kann die Uhr der Prothese korrigiert werden. Das Programm führt in vorbestimmten Abständen eine Totalspeicherung aller Daten der Prothese durch, die als Backup dient. Der Orthopädietechniker kann durch Zugriff über das Datenfernübertragungsnetz durch Eingabe einer Codenummer Informationen über den Betriebszustand der Prothese und die Prothesennutzung abfragen. Es ist die Möglichkeit vorgesehen, daß sich die Datenübertragungseinrichtung bei der Fernwirkeinheit 23 nur dann meldet, wenn das Fernwartungsprogramm Fehler im Betriebszustand der Prothese selbst erkennt.

Durch die Speicherung der Bewegungs- und Steuerdaten in der Fernwartungsvorrichtung 20, also außerhalb der Prothese, ist es möglich, im Offline Betrieb, wenn die Prothese nicht mit der Vorrichtung 20 verbunden ist, eine Gang- und/oder Aktivitätsanalyse vorzunehmen. Durch die Speicherung der Bewegungs- und/oder Steuerdaten in der Fernwartungsvorrichtung 20 ist ferner ein Datenaustausch mit der Fernwirkeinheit 23 möglich, wenn die Prcthese nach der Übergabe der aktuellen Bewegungs- und Steuerdaten von der Fernwartungsvorrichtung entfernt wird. Es ist auch eine Online-Fernwartung möglich, bei der der Prothesenträger die Prothese nicht ablegt, sondern die Steuerdaten und/oder Bewegungsdaten online während des Tragens an die Fernwirkeinheit 23 gesendet werden, der dann online eine Beurteilung und gegebenenfalls eine Korrektur vornimmt. Über die Bedien- und Anzeigekonsole ist es möglich, daß der Prothesenträger nach Anschließen der Prothese über eventuelle Fehler informiert wird und gegebenenfalls selbst eine Korrektur vornehmen kann.

Mit der vorbeschriebenen Vorrichtung und dem Verfahren zur Fernwartung ist es somit möglich, die Beinprothese einer Fernwartung zu unterziehen, ohne daß der Prothesenträger persönlich zu einem Orthopädietechniker kommen muß, um seine Prothese dort beim Gehen einstellen zu lassen.

In einer Weiterbildung ist die Fernwirkeinheit 23 so ausgebildet, daß mit ihr mehr als eine Prothese ferngewartet werden können.

## Patentansprüche

1. Vorrichtung zur Fernwartung einer elektronisch gesteuerten Prothese, wobei die Prothese eine Steuereinrichtung (11) zur Erfassung von eine Bewegung des Prothesenträger charakterisierenden Bewegungsdaten und zum Ausgeben von Steuerdaten an die Prothese aufweist,
wobei die Vorrichtung (20) zur Fernwartung eine
erste Datenübertragungseinrichtung (21), die mit der Steuereinrichtung (11) der Prothese (1) verbindbar ist, über die bidirektional Bewegungs- und/oder Steuerdaten zwischen der Steuervorrichtung (11) der Prothese und der ersten Datenübertragungsvorrichtung (21) übertragbar sind,
eine zweite Datenübertragungseinrichtung (22) zum Übertragen von Bewegungs- und/oder Steuerdaten an eine entfernt vorgesehene Fernwirkeinheit (23) und zum Empfangen von Daten von der Fernwirkeinheit (23), und
eine Speichereinrichtung (24) zum Speichern von Bewegungs- und/oder Steuerdaten aufweist, und
wobei die Fernwirkeinheit (23) mit der zweiten Datenübertragungseinrichtung (22) über ein Datenfernübertragungsnetz verbunden ist.

2. Vorrichtung nach Anspruch 1, **gekennzeichnet durch** eine Steuereinrichtung (24), die in Abhängigkeit von einem vorbestimmten Programm eine Auswertung und/oder Übertragung der Bewegungs- und/oder Steuerdaten steuert.

3. Vorrichtung nach Anspruch 1 oder 2, **dadurch gekennzeichnet, daß** die Prothese (1) eine oder mehrere aufladbare Batterien aufweist und die Vorrichtung (20) zur Fernwartung eine Einrichtung (27) zum Aufladen der Batterien, wenn die Prothese an die Vorrichtung (20) angeschlossen ist, aufweist.

4. Vorrichtung nach einem der Ansprüche 1 bis 3, **gekennzeichnet durch** eine extern steuerbare Uhr (25) zum Ermöglichen einer Synchronisation mit einer in der Prothese vorgesehenen Uhr.

5. Vorrichtung nach einem der Ansprüche 1 bis 4, **gekennzeichnet durch** eine Anzeige-/Eingabeeinheit (26) zum Anzeigen von die Prothese betreffenden Daten und zum Eingeben von Befehlen.

6. Vorrichtung nach einem der Ansprüche 1 bis 5, **dadurch gekennzeichnet, daß** die Steuereinrichtung so ausgebildet ist, daß sie eine Kommunikation über die zweite Datenübertragungsvorrichtung (22) mit der Fernwirkeinheit (23) initiiert, wenn die Bewegungs- und/oder Steuerdaten von vorgegebenen Daten abweichen.

7. Verfahren zur Fernwartung einer elektronisch ansteuerbaren Prothese mit den Schritten:
a) Erfassen von den Betrieb der Prothese charakterisierenden Daten während des Tragens der Prothese;
b) Übertragen der erfaßten Daten an eine Fernwartungsvorrichtung (20) mit einer Speicher- und Auswerteeinheit;
c) Zugreifen auf die in der Speicher- und Auswerteeinrichtung abgelegten Daten durch eine Fernwirkeinheit (23) über ein Datenfernübertragungsnetz;
d) Beurteilen und gegebenenfalls Ändern der erfaßten Daten; und
e) Übertragen der gegebenenfalls geänderten Daten an die Speicher- und Auswertevorrichtung über das Datenfernübertragungsnetz;
f) Übertragen der geänderten Daten von der Speicher- und Auswertevorrichtung (20) an die Prothese.

8. Verfahren nach Anspruch 7, **dadurch gekennzeichnet, daß** die Fernwartung über das Telephonnetz ausgeführt wird.

9. Verfahren nach Anspruch 7 oder 8, **dadurch gekennzeichnet, daß** von der Prothese Bewegungsdaten über einen vorbestimmten Zeitraum erfaßt werden und diese Bewegungsdaten in der Speicher- und Auswerteeinheit (20) ausgewertet werden und daß eine Korrektur der den Betrieb der Prothese charakterisierenden Daten anhand der Bewegungsdaten erfolgt.

10. Verfahren nach einem der Ansprüche 7 bis 9, **dadurch gekennzeichnet, daß** die Fernwartung ausgeführt wird, wenn die Prothese abgelegt ist.

## Claims

1. Apparatus for the remote maintenance of an electronically controlled prosthesis, the prosthesis having a control device (11) for capturing movement data which characterize a movement by the prosthesis wearer and for outputting control data to the prosthesis,
where the apparatus (20) for remote maintenance has
a first data transmission device (21), which can be connected to the control device (11) in the prosthesis (1) and can be used to transmit movement and/or control data bidirectionally between the control apparatus (11) in the prosthesis and the first data transmission apparatus (21),
a second data transmission device (22) for transmitting movement and/or control data to a remotely provided telecontrol unit (23) and for receiving data from the telecontrol unit (23), and
a memory device (24) for storing movement and/or control data, and
where the telecontrol unit (23) is connected to the second data transmission device (22) via a data communications network.

2. Apparatus according to Claim 1, **characterized by** a control device (24) which takes a predetermined program as a basis for controlling evaluation and/or transmission of the movement and/or control data.

3. Apparatus according to Claim 1 or 2, **characterized in that** the prosthesis (1) has one or more chargeable batteries, and the apparatus (20) for remote maintenance has a device (27) for charging the batteries when the prosthesis is connected to the apparatus (20).

4. Apparatus according to one of Claims 1 to 3, **characterized by** an externally controllable clock (25) for allowing synchronization with a clock provided in the prosthesis.

5. Apparatus according to one of Claims 1 to 4, **characterized by** a display/input unit (26) for displaying data relating to the prosthesis and for inputting commands.

6. Apparatus according to one of Claims 1 to 5, **characterized in that** the control device is designed such that it initiates communication with the telecontrol unit (23) via the second data transmission apparatus (22) if the movement and/or control data differ from prescribed data.

7. Method for the remote maintenance of an electronically activatable prosthesis, having the following steps:
a) data which characterize the operation of the prosthesis are captured while the prosthesis is being worn;
b) the captured data are transmitted to a remote-maintenance apparatus (20) having a memory and evaluation unit;
c) the data stored in the memory and evaluation device are accessed by a telecontrol unit (23) via a data communications network;
d) the captured data are assessed and possibly changed; and
e) the possibly changed data are transmitted to the memory and evaluation apparatus via the data communications network;
f) the changed data are transmitted from the memory and evaluation apparatus (20) to the prosthesis.

8. Method according to Claim 7, **characterized in that** the remote maintenance is performed via the telephone network.

9. Method according to Claim 7 or 8, **characterized in that** the prosthesis captures movement data over a predetermined period of time and these movement data are evaluated in the memory and evaluation unit (20), and **in that** data which characterize the operation of the prosthesis are corrected using the movement data.

10. Method according to one of Claims 7 to 9, **characterized in that** the remote maintenance is performed when the prosthesis is not being worn.

## Revendications

1. Dispositif de télé-entretien d'une prothèse commandée électroniquement, dans lequel la prothèse comporte un dispositif de commande (11) pour la saisie de données de mouvement caractérisant un mouvement du porteur de la prothèse et pour la transmission de données de commande à la prothèse, dans lequel le dispositif (20) de télé-entretien comporte un premier dispositif de transmission des données (21), qui peut être relié au dispositif de commande (11) de la prothèse (1), par le biais duquel les données de mouvement et/ou de commande peuvent être transmises de manière bidirectionnelle entre le dispositif de commande (11) de la prothèse et le premier dispositif de transmission des données (21), un second dispositif de transmission des données (22) pour la transmission de données de mouvement et/ou de commande à une unité de commande à distance (23) prévue à distance et pour la réception de données provenant de l'unité de commande à distance (23), et un dispositif de mémorisation (24) pour l'enregistrement de données de mouvement et/ou de commande et dans lequel l'unité de commande à distance (23) est reliée au deuxième dispositif de transmission des données (22) par le biais d'un réseau de télétransmission des données.

2. Dispositif selon la revendication 1, **caractérisé par** un dispositif de commande (24) qui commande une analyse et/ou une transmission des données de mouvement et/ou de commande en fonction d'un programme prédéterminé.

3. Dispositif selon la revendication 1 ou 2, **caractérisé en ce que** la prothèse (1) comprend une ou plusieurs piles rechargeables et le dispositif (20) de télé-entretien comporte un dispositif (27) de recharge des piles lorsque la prothèse est raccordée au dispositif (20).

4. Dispositif selon l'une quelconque des revendications 1 à 3, **caractérisé par** une horloge (25) pouvant être commandée de l'extérieur pour permettre une synchronisation avec une horloge prévue dans la prothèse.

5. Dispositif selon l'une quelconque des revendications 1 à 4, **caractérisé par** une unité d'affichage/d'entrée (26) pour l'affichage des données concernant la prothèse et pour l'entrée de commandes.

6. Dispositif selon l'une quelconque des revendications 1 à 5, **caractérisé en ce que** le dispositif de commande est configuré de telle sorte qu'il amorce une communication avec l'unité de commande à distance (23) par le biais du second dispositif de transmission des données (22) lorsque les données de mouvement et/ou de commande diffèrent des données prédéfinies.

7. Procédé de télé-entretien d'une prothèse pouvant être commandée électroniquement avec les étapes suivantes :
a) saisie des données caractérisant le fonctionnement de la prothèse, pendant le port de la prothèse,
b) transmission des données saisies à un dispositif de télé-entretien (20) avec une unité de mémorisation et d'analyse,
c) accès aux données stockées dans le dispositif de mémorisation et d'analyse au moyen d'une unité de commande à distance (23) par le biais d'un réseau de télétransmission des données,
d) évaluation et le cas échéant modification des données saisies et
e) transmission des données éventuellement modifiées au dispositif de mémorisation et d'analyse par le biais du réseau de télétransmission des données,
f) transmission des données modifiées du dispositif de mémorisation et d'analyse (20) à la prothèse.

8. Procédé selon la revendication 7, **caractérisé en ce que** le télé-entretien est effectué par le biais du réseau téléphonique.

9. Procédé selon la revendication 7 ou 8, **caractérisé en ce que** les données de mouvement de la prothèse sont saisies dans un laps de temps prédéterminé et que ces données de mouvement sont analysées dans l'unité de mémorisation et d'analyse (20) et **en ce qu'**une correction des données caractérisant le fonctionnement de la prothèse se fait à l'aide des données de mouvement.

10. Procédé selon l'une des revendications 7 à 9, **caractérisé en ce que** le télé-entretien est effectué lorsque la prothèse a été ôtée.
